# EUROPÄISCHE PATENTANMELDUNG

(11) **EP 1 064 885 A1**
(43) Veröffentlichungstag der Anmeldung: **03.01.2001**
(21) Anmeldenummer: 00810491.1
(22) Anmeldetag: 06.06.2000
(51) Int. Cl.: A61B 17/70

(54) **Haltevorrichtung für die Wirbelsäule**

(30) Priorität: 02.07.1999 EP 99810579
(71) Anmelder: Sulzer Orthopedics Ltd., 6340 Baar (CH)
(72) Erfinder: Ventre, Carlo, 8488 Turbenthal (CH); Donno, Cosimo, 8400 Winterthur (CH); Studer, Armin, 6312 Steinhausen (CH); Adali, Onur, 8317 Tagelswangen (CH); Lobsiger, André, 8405 Winterthur (CH); Hug, Daniel, 8484 Weisslingen (CH); Berani, Raffael, 8610 Uster (CH); Ambühl, Daniel, 8424 Embrach (CH)
(74) Vertreter: Sulzer Management AG

(57) **Zusammenfassung**

Eine Haltevorrichtung für die Wirbelsäule weist Mittel zur Befestigung an einem Wirbel auf. Ferner weist sie eine Aufnahme (2) für einen Stab (3) auf, die an der den Befestigungsmitteln abgewandten Seite der Haltevorrichtung vorgesehen ist. Weiterhin weist die Haltevorrichtung Mitteln (5) auf zum Herstellen einer starren Verbindung der Haltevorrichtung mit dem in der Aufnahme befindlichen Stab (3). Schliesslich weist die Vorrichtung Mittel (4,20;6a,22a,62a;6b,22b,62;6c,21 c,61 c;6d,22d,62d; 21e,22e,24e;21f,22f,24f;6g,21g,61g;6h;6i;6j;6k) zum Zurückhalten des Stabes in der Aufnahme (2,2a,2b,2c,2d,2e,2f,2g) auf, welche so ausgebildet und mit der Haltevorrichtung derart in Verbindung bringbar sind, dass sie einen in die Aufnahme (2,2a,2b,2c,2d,2e,2f,2g) eingebrachten Stab (3,3a,3b,3c,3d,3e,3f,3g) vorläufig in der Aufnahme zurückhalten, bis die Haltevorrichtung mit Hilfe der Mittel zum Herstellen der starren Verbindung an dem Stab fixiert ist.

## Beschreibung

Die Erfindung betrifft eine Haltevorrichtung für die Wirbelsäule gemäss dem Oberbegriff des unabhängigen Patentanspruchs.

Bei Deformationen der Wirbelsäule des Menschen kommen derartige Haltevorrichtungen zum Einsatz. Vom Prinzip her werden die Haltevorrichtungen an mehreren Wirbeln befestigt und mittels einer oder mehrerer Stäbe miteinander verbunden, wodurch die Wirbelsäule mechanisch in eine Sollposition gezwungen wird.

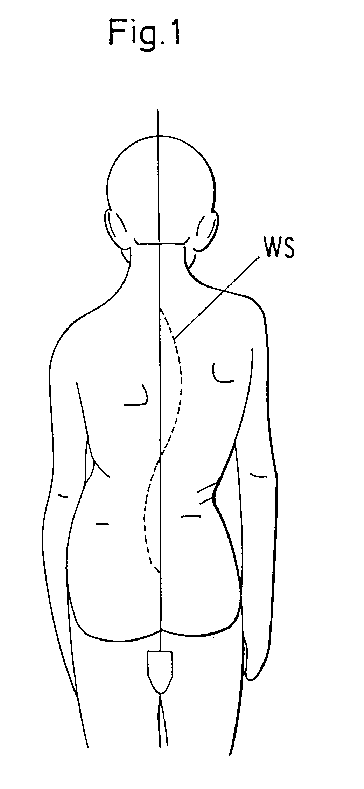

Skoliose ist beispielsweise eine solche Deformation der Wirbelsäule. Als Skoliose wird eine seitliche (laterale) Verbiegung der Wirbelsäule bezeichnet, die auch noch mit einer Drehung (Torsion) der einzelnen Wirbel verbunden sein kann. Man unterscheidet prinzipiell zwischen einer totalen oder C-förmigen Skoliose (Krümmung der Wirbelsäule nach einer Seite ohne Gegenkrümmung), einer zusammengesetzten oder S-förmigen Skoliose (Krümmung mit Gegenkrümmung) und einer Trippelskoliose (Krümmung mit kompensatorischer Gegenkrümmung nach kranial und kaudal). Eine zusammengesetzte oder S-förmige Skoliose ist beispielsweise in Fig. 1 schematisch angedeutet. Die gestrichelte Linie deutet den S-förmigen Verlauf der Wirbelsäule WS an.

Beim operativen Richten der Wirbelsäule werden Haltevorrichtungen, die z.B. einen Haken aufweisen, an den Wirbeln befestigt. Die Haken greifen unter bzw. hinter die Lamina des jeweiligen Wirbels und verhaken sich dort, sie können aber auch an den Pedikeln angreifen bzw. an den jeweiligen Prozessus transversus (Dornfortsatz). Typischerweise wird zunächst eine Haltevorrichtung an je einem Wirbel an den beiden Enden des Bereichs der Deformation der Wirbelsäule befestigt. Die Haltevorrichtungen weisen an ihrem dem Haken abgewandten Ende eine Aufnahme für einen Stab auf. Der Stab weist eine zuvor festgelegte Form auf, welche bewirkt, dass dann, wenn die einzelnen Wirbel im Bereich der Deformation mittels einer Haltevorrichtung mit dem Stab verbunden sind, die Wirbelsäule einen gewünschten Verlauf aufweist.

An den Wirbeln im Bereich der Deformation der Wirbelsäule werden dann ebenfalls Haltevorrichtungen befestigt. Nach dem Befestigen einer solchen Haltevorrichtung am jeweiligen Wirbel im Bereich der Deformation wird die Haltevorrichtung mitsamt dem Wirbel zum Stab hin gezogen bis die Aufnahme der Haltevorrichtung dann den Stab aufnimmt. Sodann befindet sich der jeweilige Wirbel in seiner Sollposition. Die Haltevorrichtung muss nun mit dem Stab fest verbunden werden, denn die Wirbelsäule versucht natürlich, wieder in die Stellung der Deformation zurückzugelangen. Dies ist für den operierenden Arzt einigermassen schwierig zu bewältigen, weil er einerseits eine laterale Bewegung des Wirbels zusammen mit der daran befestigten Haltevorrichtung zum Stab hin durchführen muss, andererseits möglicherweise auch noch in dorsaler oder ventraler Richtung eine Bewegung des Wirbels mit der Haltevorrichtung bewirken muss, und schliesslich möglicherweise auch noch eine Längsverschiebung des Wirbels mitsamt der Haltevorrichtung entlang des Stabs (also praktisch eine Verschiebung nach kranial bzw. kaudal) durchführen muss. Hat er diese Bewegung des Wirbels mitsamt der daran befestigten Haltevorrichtung durchgeführt, so muss er einerseits den Wirbel in der gewünschten Position halten, andererseits gleichzeitig die Haltevorrichtung an dem Stab fixieren. Dies ist vom Handling her für den operierenden Arzt mit den oben beschriebenen Schwierigkeiten verbunden, weil eben die Wirbelsäule versucht, den ursprünglichen (deformierten) Zustand wiederherzustellen.

Es ist daher eine Aufgabe der Erfindung, eine Haltevorrichtung vorzuschlagen, welche dem operierenden Arzt das Handling beim Befestigen der Haltevorrichtung an dem Stab erleichtert.

Dies Aufgabe wird mit einer Haltevorrichtung, wie sie durch die Merkmale des unabhängigen Patentanspruchs charakterisiert ist, gelöst. Besonders vorteilhafte Ausgestaltungen ergeben sich aus den abhängigen Patentansprüchen bzw. aus der nachfolgenden Beschreibung der in den Zeichnungen dargestellten Ausführungsbeispiele.

Die erfindungsgemässe Haltevorrichtung weist Mittel zum Zurückhalten des Stabes in der Aufnahme auf, welche so ausgebildet und mit der Haltevorrichtung derart in Verbindung bringbar sind, dass sie einen in die Aufnahme eingebrachten Stab vorläufig in der Aufnahme zurückhalten, bis die Haltevorrichtung mit Hilfe der Mittel zum Herstellen der starren Verbindung an dem Stab fixiert ist. Das heisst, dass der operierende Arzt auf einfache Weise eine vorläufige Fixierung der Haltevorrichtung und damit des entsprechenden Wirbels erreichen kann, obwohl die Wirbelsäule versucht, den Zustand der Deformation wiederherzustellen. Gleichwohl bleibt die Haltevorrichtung in axialer Richtung des Stabes auf diesem verschiebbar.

Der operierende Arzt kann also zunächst einen Wirbel aus dem Bereich, in welchem die Deformation der Wirbelsäule vorliegt, in die gewünschte Position bringen und dann die Haltevorrichtung starr mit dem Stab verbinden, sodass zunächst ein Wirbel bzw. die daran befestigte Haltevorrichtung starr mit dem Stab verbunden ist. Alternativ kann der operierende Arzt auch zunächst mehrere Wirbel aus dem Bereich, in welchem die Deformation der Wirbelsäule vorliegt, vorläufig an dem Stab befestigen, sodass diese noch in axialer Richtung auf dem Stab verschiebbar sind, und erst dann die endgültigen Positionen der jeweiligen Wirbel festlegen. Dies hat den Vorteil, dass Haltevorrichtungen und die daran befestigten Wirbel relativ zueinander noch "längsverschieblich" sind, sodass erst nach dem Richten eines ganzen Bereichs der Wirbelsäule die endgültige Position der Haltevorrichtungen und der daran befestigten Wirbel festgelegt werden muss. Dies erfolgt in gleicher Weise dann durch die starre Verbindung der Haltevorrichtung mit dem Stab.

Vorteilhafterweise ist die Aufnahme für den Stab so ausgebildet, dass die Mittel zum Zurückhalten des Stabs mit der Aufnahme selbst in Verbindung bringbar sind. Dieser Bereich, also die Aufnahme selbst, ist nach dem Befestigen der Haltevorrichtung am Wirbel (z.B. mittels eines Hakens oder einer Pedikelschraube) bei der Operation für den Arzt noch am besten zugänglich.

Weiterhin können die Mittel zum Zurückhalten des Stabs vorteilhafterweise so ausgebildet sein, dass sie eine Klemmschraube aufnehmen, welche die Mittel zum Herstellen der starren Verbindung der Haltevorrichtung mit dem Stab bilden. Diese Art der starren Fixierung der Haltevorrichtung am Stab ist einfach und ohne grossen Aufwand realisierbar und resultiert in einer zuverlässigen Verbindung der Haltevorrichtung mit dem Stab.

Vorteilhafterweise können die Aufnahme für den Stab und die Mittel zum Herstellen der starren Verbindung der Haltevorrichtung mit dem Stab so ausgebildet sein, dass sie eine Dreipunktauflage bilden. Diese Art der Auflage ist wohldefiniert und besonders zuverlässig.

Für die vorläufige Fixierung bieten sich in vorteilhafter Weise mehrere Varianten an. Eine Variante sieht zum Beispiel vor, dass die Mittel zum Zurückhalten des Stabs ein Element umfassen, welches mit der Haltevorrichtung oder mit dem Stab eine Schnappverbindung eingeht.

Eine andere Variante sieht vor, dass die Mittel zum Zurückhalten des Stabs einen Deckel umfassen, welcher so ausgebildet ist, dass er mit entsprechenden Mitteln an der Haltevorrichtung eine Verbindung nach Art eines Bajonettverschlusses eingeht.

Wieder eine andere Variante sieht vor, dass die Mittel zum Zurückhalten des Stabs einen Deckel umfassen, welcher in axialer Richtung auf die Aufnahme aufschiebbar ist und welcher eine durchgehende Gewindebohrung aufweist, durch welche eine Klemmschraube hindurch schraubbar ist, und dass die Aufnahme in demjenigen Endbereich ihrer Wandung, welche dem Deckel zugewandt ist, elastisch deformierbar ausgebildet ist. Dort weist die Wandung Vorsprünge auf, welche nach einer Deformation mit entsprechend am Deckel ausgebildeten Vorsprüngen in Eingriff ist. Der Stab wird bei dieser Variante in der Aufnahme eingeschnappt, sodass er nicht unbeabsichtigt entweichen kann.

Wiederum eine andere Variante sieht vor, dass die Mittel zum Zurückhalten des Stabs einen sichelförmigen Finger umfassen, welcher in einer Aussparung zwischen zwei an der Aufnahme hakenartig ausgebildeten, feststehenden Fingern schwenkbar angeordnet ist. An der jeweiligen auf den beweglichen Finger zuweisenden Fläche der feststehenden hakenartigen Finger ist jeweils ein auf den beweglichen Finger zuweisender Zapfen vorgesehen. Dieser Zapfen greift in eine entsprechende Aussparung ein, welche an dem beweglichen Finger in der jeweiligen auf den feststehenden Finger zuweisenden Fläche vorgesehen ist. Der bewegliche Finger weist dabei einen nach aussen über die Aussparung hinaus stehenden Fortsatz auf, der mit einem Gewinde versehen ist, auf welches eine Mutter aufschraubbar ist. Somit kann durch Verschwenken des Fingers zunächst der Stab vorläufig in der Aufnahme zurückgehalten werden. Anschliessend kann durch ein Anziehen der Mutter die endgültige starre Verbindung der Haltevorrichtung mit der Stange bewirkt werden.

Weitere besonders vorteilhafte Ausgestaltungen ergeben sich aus der Beschreibung der nachfolgenden Ausführungsbeispiele einer erfindungsgemässen Haltevorrichtung. Es zeigen, teilweise in schematischer Darstellung und/oder im Schnitt:
- Fig. 1: eine Darstellung einer zusammengesetzten oder S-förmigen Skoliose,
- Fig. 2-4: ein erstes Ausführungsbeispiel einer erfindungsgemässen Haltevorrichtung, und zwar

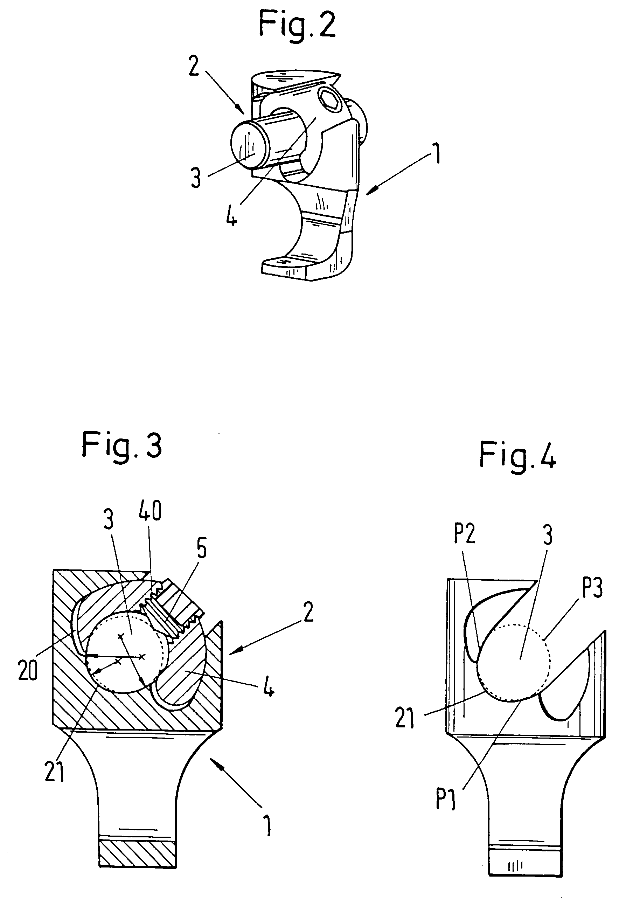

- Fig. 2: eine perspektivische Ansicht dieser Haltevorrichtung im Zusammenbau
- Fig. 3: eine Schnittdarstellung dieser Haltevorrichtung im Zusammenbau
- Fig. 4: eine schematische Darstellung zur Erläuterung der Dreipunktauflage

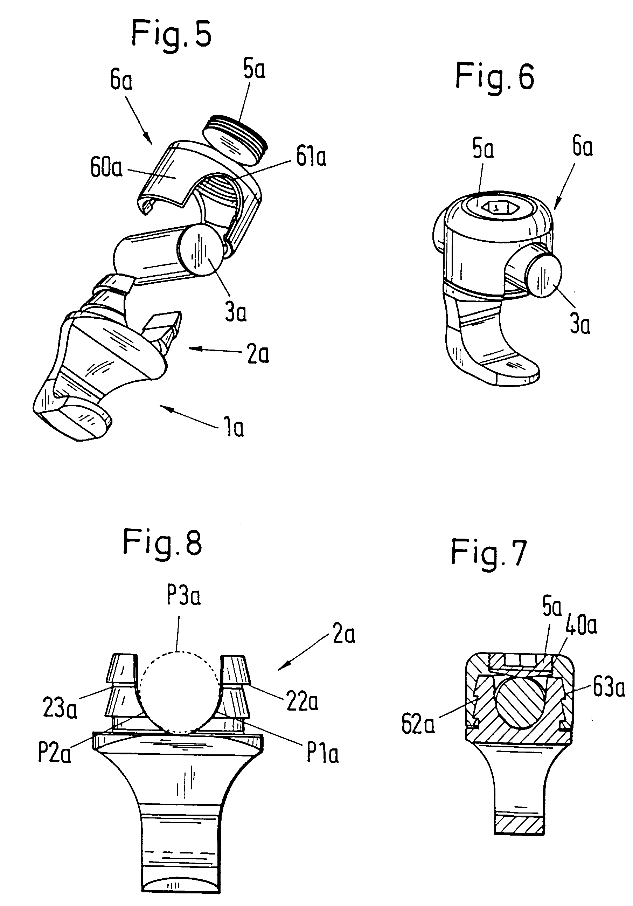

- Fig. 5-8: ein zweites Ausführungsbeispiel einer erfindungsgemässen Haltevorrichtung, und zwar
- Fig. 5: eine perspektivische Explosionsdarstellung dieser Haltevorrichtung,
- Fig. 6: eine perspektivische Darstellung dieser Haltevorrichtung im Zusammenbau,
- Fig. 7: eine Schnittdarstellung dieser Haltevorrichtung im Zusammenbau
- Fig. 8: eine schematische Darstellung zur Erläuterung der Dreipunktauflage

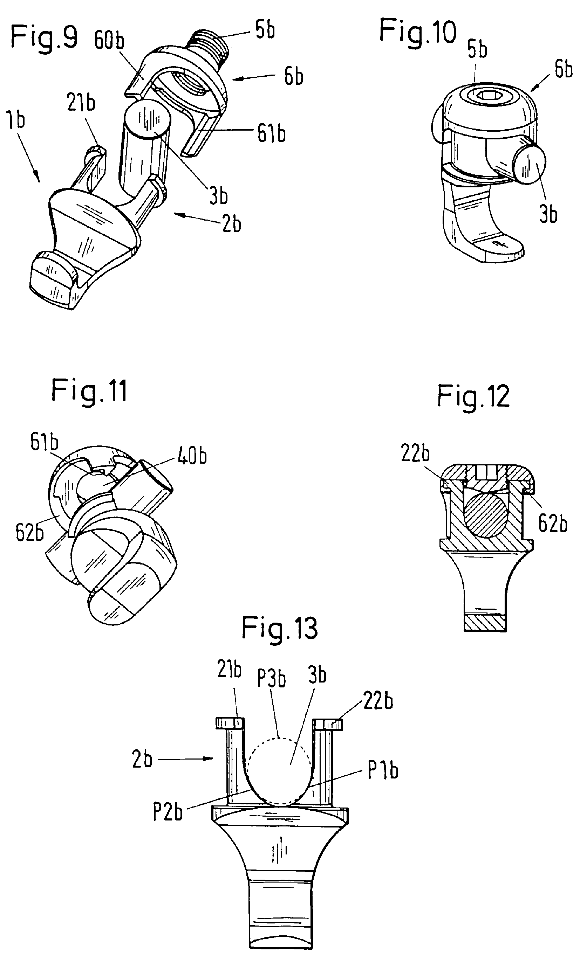

- Fig. 9-13: ein drittes Ausführungsbeispiel einer erfindungsgemässen Haltevorrichtung, und zwar
- Fig. 9: eine perspektivische Explosionsdarstellung dieser Haltevorrichtung,
- Fig. 10: eine perspektivische Darstellung dieser Haltevorrichtung im Zusammenbau,
- Fig. 11: eine perspektivische Explosionsdarstellung dieser Haltevorrichtung in einer Ansicht von unten,
- Fig. 12: eine Schnittdarstellung dieser Haltevorrichtung im Zusammenbau,
- Fig. 13: eine schematische Darstellung zur Erläuterung der Dreipunktauflage,

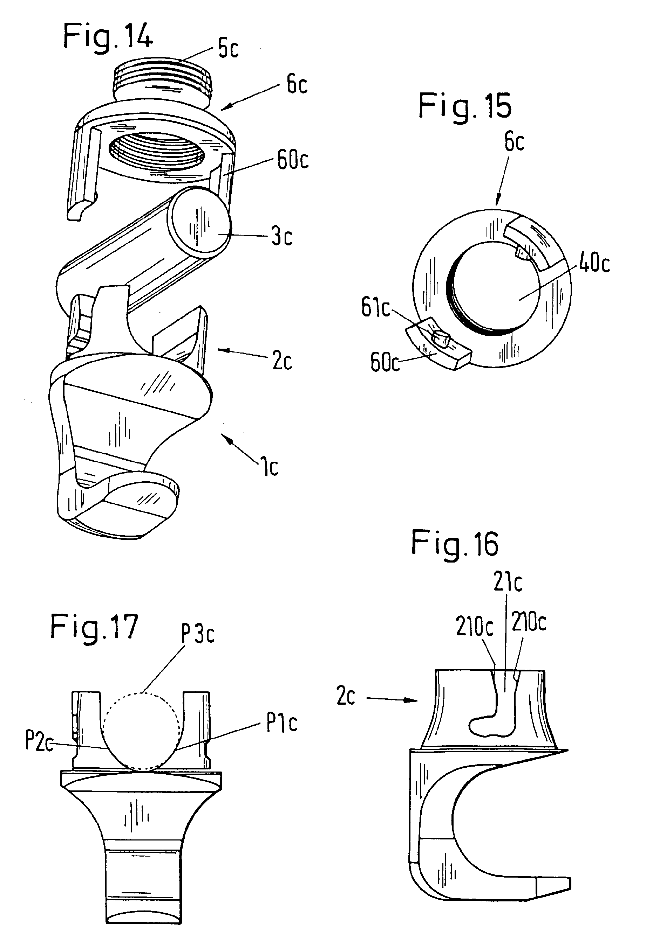

- Fig. 14-17: ein viertes Ausführungsbeispiel einer erfindungsgemässen Haltevorrichtung, und zwar
- Fig. 14: eine perspektivische Explosionsdarstellung dieser Haltevorrichtung,
- Fig. 15: eine perspektivische Ansicht des Deckels dieser Haltevorrichtung von unten,
- Fig. 16: eine perspektivische Ansicht dieser Haltevorrichtung ohne Deckel von der Seite,
- Fig. 17: eine schematische Darstellung zur Erläuterung der Dreipunktauflage,

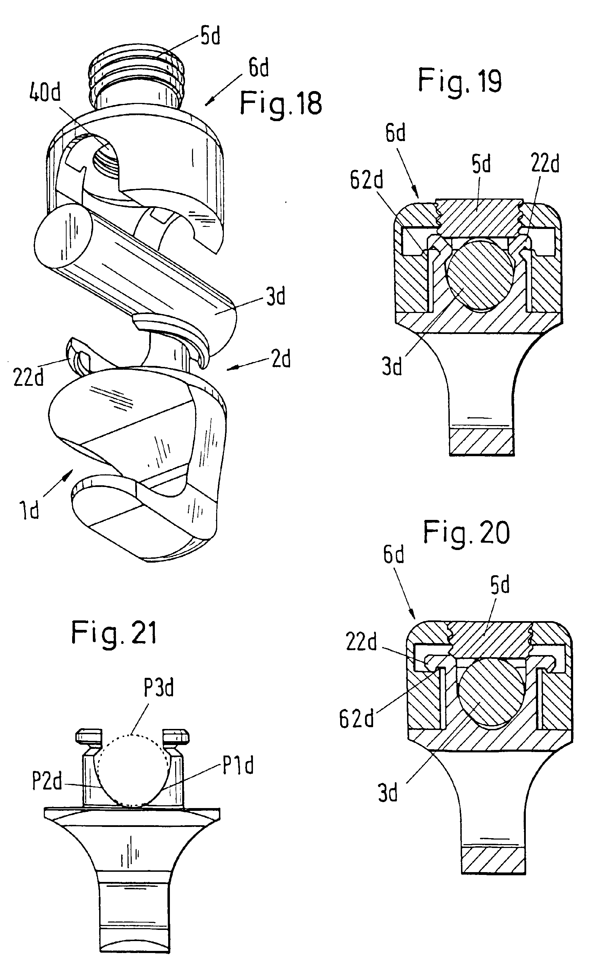

- Fig. 18-21: ein fünftes Ausführungsbeispiel einer erfindungsgemässen Haltevorrichtung, und zwar
- Fig. 18: eine perspektivische Explosionsdarstellung dieser Haltevorrichtung,
- Fig. 19: eine Schnittdarstellung dieser Haltevorrichtung mit nur leicht eingeschraubter Klemmschraube,
- Fig. 20: eine Schnittdarstellung dieser Haltevorrichtung mit vollständig eingeschraubter Klemmschraube,
- Fig. 21: eine schematische Darstellung zur Erläuterung der Dreipunktauflage,

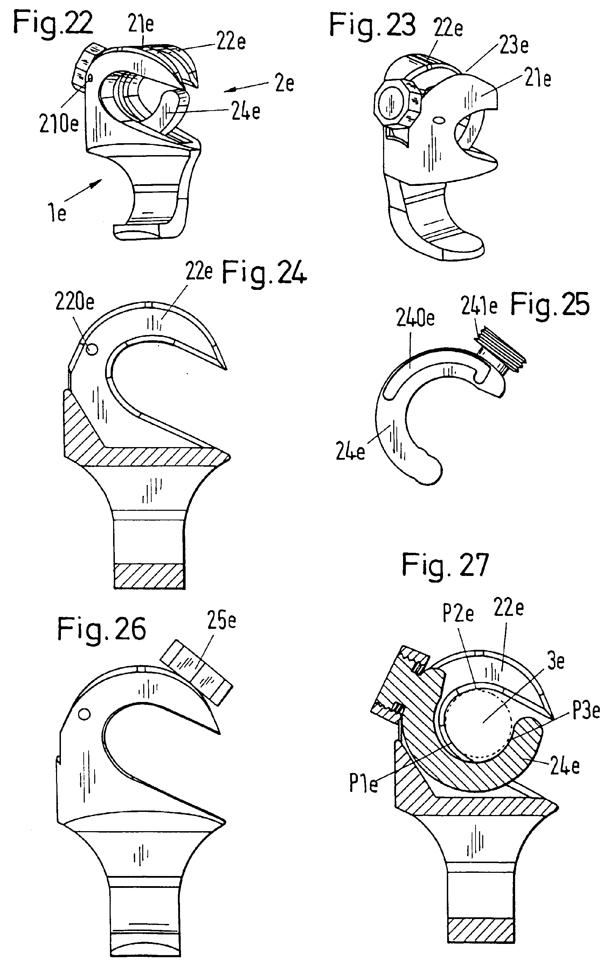

- Fig. 22-27: ein sechstes Ausführungsbeispiel einer erfindungsgemässen Haltevorrichtung, und zwar
- Fig. 22: eine perspektivische Ansicht dieser Haltevorrichtung von vorne, im Zusammenbau,
- Fig. 23: eine perspektivische Ansicht dieser Haltevorrichtung von seitlich vorne, im Zusammenbau,
- Fig. 24: eine Schnittdarstellung dieser Haltevorrichtung ohne den beweglichen Finger,
- Fig. 25: den beweglichen Finger in einer Seitenansicht,
- Fig. 26: eine Ansicht dieser Haltevorrichtung von vorne, mit ausgeschwenktem Finger,
- Fig. 27: eine Schnittdarstellung dieser Haltevorrichtung mit eingeschwenktem Finger, zur Erläuterung der Dreipunktauflage,

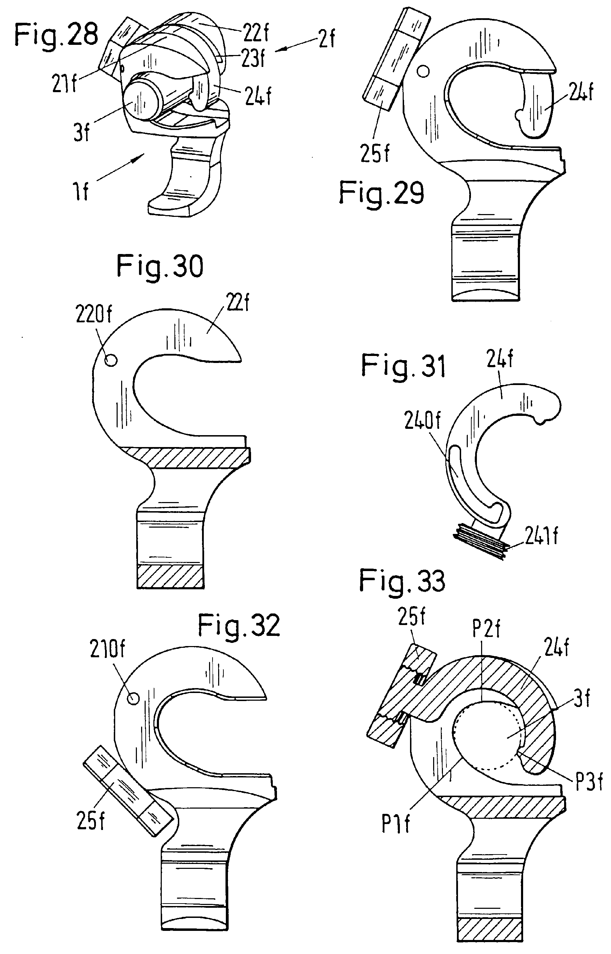

- Fig. 28-33: ein siebtes Ausführungsbeispiel einer erfindungsgemässen Haltevorrichtung, und zwar
- Fig. 28: eine perspektivische Ansicht dieser Haltevorrichtung von vorne, im Zusammenbau,
- Fig. 29: eine Ansicht dieser Haltevorrichtung von vorne, mit eingeschwenktem beweglichen Finger,
- Fig. 30: eine Schnittdarstellung dieser Haltevorrichtung, ohne den beweglichen Finger,
- Fig. 31: den beweglichen Finger in einer Seitenansicht,
- Fig. 32: eine Ansicht dieser Haltevorrichtung von vorne, mit ausgeschwenktem beweglichem Finger,
- Fig. 33: eine Schnittdarstellung dieser Haltevorrichtung, mit eingeschwenktem Finger, zur Erläuterung der Dreipunktauflage,

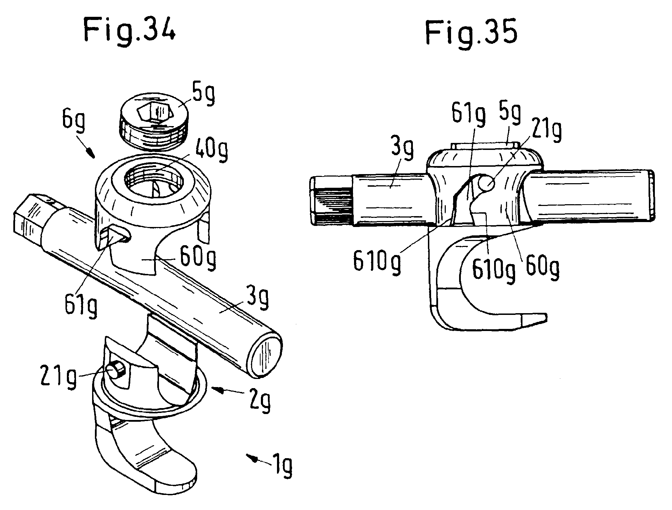

- Fig. 34: ein achtes Ausführungsbeispiel einer erfindungsgemässen Haltevorrichtung in Explosionsdarstellung,
- Fig. 35: das Ausführungsbeispiel gemäss Fig. 34 in Seitenansicht,

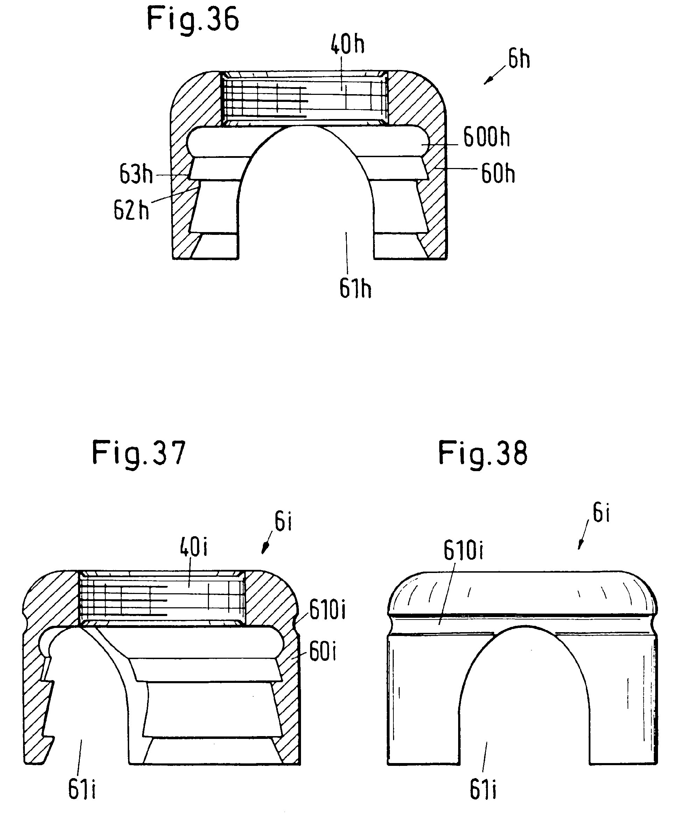

- Fig. 36: ein Ausführungsbeispiel des Deckels einer erfindungsgemässen Haltevorrichtung in Schnittdarstellung,
- Fig. 37: ein weiteres Ausführungsbeispiel des Deckels einer erfindungsgemässen Haltevorrichtung in Schnittdarstellung,
- Fig. 38: das Ausführungsbeispiel des Deckels gemäss Fig. 37 in einer Ansicht von vorne,

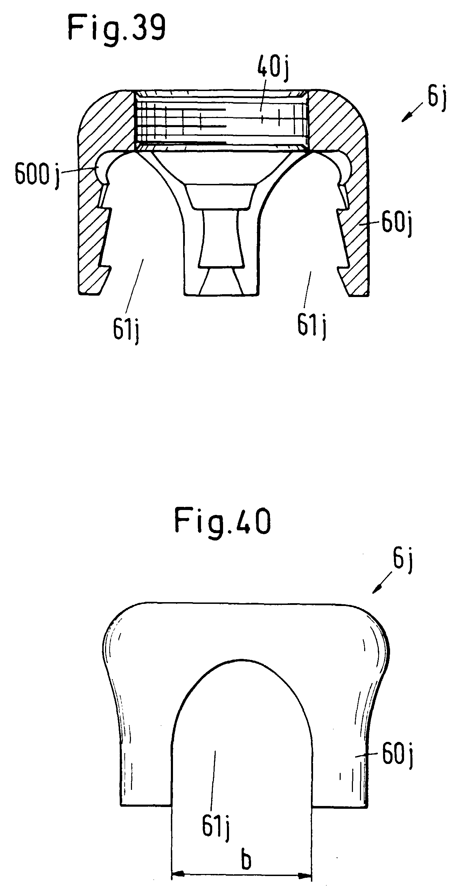

- Fig. 39: ein weiteres Ausführungsbeispiel des Deckels einer erfindungsgemässen Haltevorrichtung in Schnittdarstellung,
- Fig. 40: das Ausführungsbeispiel des Deckels gemäss Fig. 39 in einer Ansicht von vorne,

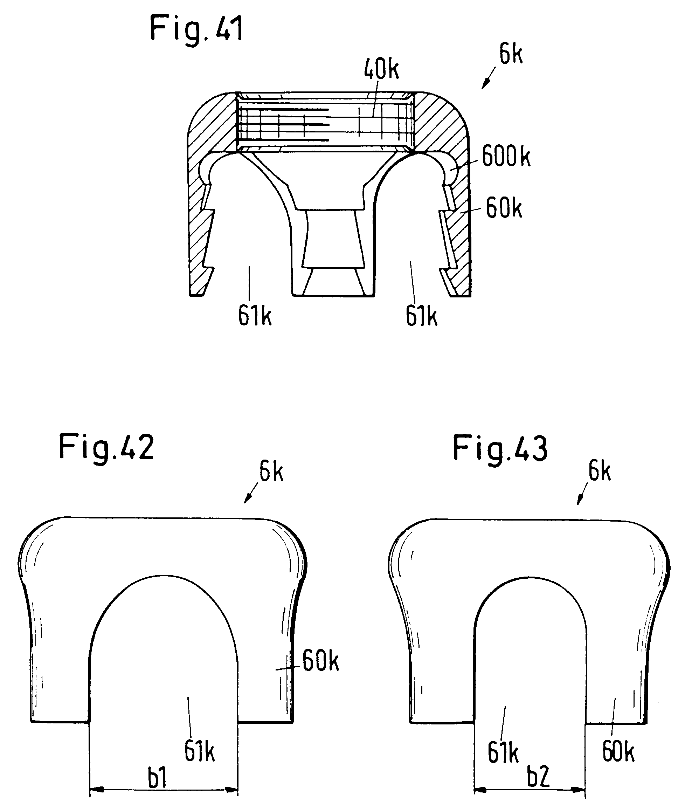

- Fig. 41: ein weiteres Ausführungsbeispiel des Deckels einer erfindungsgemässen Haltevorrichtung in Schnittdarstellung,
- Fig. 42: das Ausführungsbeispiel des Deckels gemäss Fig. 41 in einer Ansicht von vorne
und
- Fig. 43: das Ausführungsbeispiel des Deckels gemäss Fig. 41 in einer Seitenansicht.

Wenn im folgenden nur hakenartige Ausführungsbeispiele für die Befestigung der Haltevorrichtung am Wirbel gezeigt sind, so gelten die Ausführungen sinngemäss natürlich auch für anderen Arten der Befestigung der Haltevorrichtung, wie z.B. für Befestigungen mittels (Pedikel-)Schrauben. Im übrigen können die einzelnen Haltevorrichtungen, die nachfolgend für posteriore Zugänge zur Wirbelsäule beschrieben sind, natürlich auch so modifiziert sein, dass sie für anteriore Zugänge einsetzbar sind.

In den Figuren 2 bis 4 ist ein erstes Ausführungsbeispiel einer erfindungsgemässen Haltevorrichtung gezeigt. Insbesondere erkennt man hier einen Hakengrundkörper 1, der im oberen Teil eine Aufnahme 2 aufweist, welche zur Aufnahme eines Stabes 3 dient (in den Figuren ist jeweils nur ein Ausschnitt des Stabs dargestellt). Ferner erkennt man einen zylindrischen, im Querschnitt sichelförmigen Clip 4, welcher so ausgebildet ist, dass er mit dem Stab 3 eine Schnappverbindung eingeht. Dazu reicht der Clip 4 um mehr als den halben Umfang um den Stab 3 herum und ist bis zu einem gewissen Grade elastisch. Die Aufnahme 2 weist eine entsprechende zylindrische, im wesentlichen sichelförmige Aussparung 20 auf, welche geringfügig grösser ist als der Clip 4, sodass der auf den Stab aufgeschnappte Clip 4 in die Aussparung 20 in der Aufnahme 2 hinein eingeführt werden kann, also hineingeschoben werden kann, bzw. die Haltevorrichtung über den Clip 4 drüber geschoben werden kann. Ferner weist die Aufnahme 2 noch eine Aussparung 21 auf, in welcher der Stab 3 zu liegen kommt.

Ist der Clip 4 einmal in die Aussparung 20 der Aufnahme 2 eingeführt bzw. die Haltevorrichtung über den auf den Stab 3 aufgeschnappten Clip 4 drüber geschoben, so kann der Stab 3 nicht mehr aus der Aussparung 20 bzw. 21 entweichen, auch wenn die Wirbelsäule eine derartige Rückstellbewegung zu bewirken versucht. Es ist eine vorläufige Fixierung erfolgt, die allerdings noch ein Verschieben der Haltevorrichtung auf der Stange in Richtung des Stabs 3 gestattet.

Der Clip 4 ist ferner mit einer durchgehenden Gewindebohrung 40 für eine Klemmschraube 5 versehen. Wenn diese Klemmschraube 5 durch die Gewindebohrung 40 geschraubt wird, so drückt sie den Stab 3 fest in die Aussparung 21 hinein, während der sichelförmige Clip 4 an die Innenwand der sichelförmigen Aussparung 20 der Aufnahme 2 angepresst wird. Ist dies erfolgt, so ist die Haltevorrichtung nicht mehr verschiebbar, sondern starr mit dem Stab 3 verbunden.

Dabei sind die einzelnen Elemente hier so ausgebildet, dass es zu einer Dreipunktauflage an den Punkten P1,P2,P3 kommt. Bei diesem Ausführungsbeispiel kommt ein Auflagepunkt P1 in dem Bereich der Aussparung 21 zu liegen, während ein weiterer Auflagepunkt P2 in dem Bereich des sichelförmigen Clips 4 zu liegen kommt. Der dritte Auflagepunkt P3 wird durch die Klemmschraube 5 gebildet. Solche Dreipunktauflagen sind an sich bekannt und bewirken ein sicheres Aufliegen des Stabs 3.

In den Figuren 5-8 ist ein weiteres Ausführungsbeispiel einer erfindungsgemässen Haltevorrichtung zu erkennen. Aus der Explosionsdarstellung in Fig. 5 erkennt man den Hakengrundkörper 1a mit der Aufnahme 2a, den Stab 3a, sowie einen Deckel 6a. Der Deckel 6a weist einen umlaufenden Flansch 60a auf, welcher vom Deckel absteht. In dem Flansch sind zwei Aussparungen 61a für den Stab 3a vorgesehen. Auf der Innenwand des Flanschs 60a sind Vorsprünge 62a und Vertiefungen 63a vorgesehen, welche mit entsprechenden Vorsprüngen 22a und Vertiefungen 23a auf der Aussenwand der Aufnahme 2a eine Schnappverbindung eingehen. Bei der Montage des Deckels 6a kann der untere Vorsprung im Deckel beispielsweise zunächst nur über den oberen Vorsprung auf der Aussenwand der Aufnahme geschoben werden, sodass er in dieser Stellung einschnappt. Bereits dann ist der Stab 3a schon so gefangen, dass er nicht mehr nach oben aus der Aufnahme entweichen kann. Gleichzeitig ist natürlich die Haltevorrichtung (und damit natürlich auch der Wirbel, mit welchem die Haltevorrichtung verbunden ist) noch in Richtung des Stabs 3a beweglich und auch noch ein wenig in axialer Richtung verkippbar, was unter Umständen das Handling für den operierenden Arzt erleichtert. Alternativ kann der untere Vorsprung im Deckel 6a auch über den unteren Vorsprung auf der Aussenwand der Aufnahme geschoben werden, was in einer geringeren axialen Verkippbarkeit resultiert, aber immer noch die Verschiebbarkeit der Haltevorrichtung relativ zum Stab 3a gewährleistet.

Schliesslich wird die Klemmschraube 5a durch die im Deckel 6a vorgesehene Gewindebohrung 40a geschraubt. Sobald die Klemmschraube 5a sich auf dem Stab 3a abstützt, zieht sie den Deckel 6a nach oben, wobei sich dann die Vorsprünge 62a und Vertiefungen 63a auf den entsprechenden Vorsprüngen 22a und 23a der Aufnahme 2a abstützen. Auf diese Weise wird dann die Haltevorrichtung starr mit dem Stab 3a verbunden (Fig. 6, Fig. 7).

In Fig. 8 erkennt man schliesslich noch die Dreipunktauflage an den Auflagepunkten P1a,P2a,P3a. Eine derartige Dreipunktauflage ist konventionell, dazu muss die Ausnehmung 21a in der Aufnahme 2a in dem unteren Bereich einen Radius aufweisen, der kleiner ist als der Radius des Stabs 3a. In den an diesen Bereich anschliessenden Bereichen muss der Radius der Ausnehmung dann grösser sein als der Radius des Stabs 3a. Dies erfolgt vorzugsweise mit einem tangentialen Übergang. Diese Art der Dreipunktauflage ist aber bereits für sich genommen bekannt.

Den Ausführungsbeispielen gemäss den Figuren 2 bis 4 und denen gemäss den Figuren 5 bis 8 ist gemeinsam, dass sie zum vorläufigen Zurückhalten des Stabs 3 bzw. 3a ein Element umfassen, welches mit dem Stab oder mit der Haltevorrichtung eine Schnappverbindung eingeht. Zu dieser Art der Verbindung gibt es natürlich Alternativen, wie im folgenden noch beschrieben wird.

In den Figuren 9 bis 13 ist ein weiteres Ausführungsbeispiel der erfindungsgemässen Haltevorrichtung dargestellt. Zu erkennen sind ein Hakengrundkörper 1b mit einer Aufnahme 2b, in welcher der Stab 3b zu liegen kommt. Ferner erkennt man einen aufschiebbaren Deckel 6b, welcher nach dem Aufschieben nach der Art eines Bajonettverschlusses eine Verbindung eingeht. Hierzu weist der Deckel 6b zwei vom Deckel abstehende Flansche 60b auf, die jeweils eine Anlagefläche 61b aufweisen, welche mit einer entsprechenden Anlagefläche 21b zusammenwirkt, sodass beim Aufschieben des Deckels 6b die Anlagefläche 21b an dem jeweiligen Flansch 61b an der jeweiligen Anlagefläche 21b an der Aufnahme 2b entlang geführt ist. Der Deckel 6b weist ferner an demjenigen Ende der Anlagefläche 61b, welches der inneren Stirnfläche des Deckels zugewandt ist, einen in Umfangsrichtung verlaufenden Vorsprung 62b auf. Entsprechend ist ein von der Aufnahme 2b nach aussen abstehender Vorsprung 22b am oberen Ende der Aufnahme 2b vorgesehen. Ist der Deckel 6b bis zum Ende der Anlagefläche 61b an der Anlagefläche 21b entlang auf die Aufnahme 2b aufgeschoben worden, so kann er gegenüber dem Hakengrundkörper 1b bzw. der Aufnahme 2b verdreht werden. Dabei hintergreift der Vorsprung 62b des Deckels 6b den nach aussen von der Aufnahme 2b abstehenden Vorsprung 22b nach Art eines Bajonettverschlusses. Sodann ist der Stab 3b gefangen und kann nicht mehr nach oben aus der Aufnahme entweichen, gleichzeitig bleibt die Haltevorrichtung aber noch in axialer Richtung auf dem Stab 3a verschiebbar. Die endgültige starre Fixierung der Haltevorrichtung am Stab 3b erfolgt derart, dass die durch die Gewindebohrung 40b durchgeführte Klemmschraube 5b den Stab 3b in die Aufnahme hineindrückt, und dabei den Deckel 6b nach oben zieht. Dieser stützt sich über die zusammenwirkenden Vorsprünge 62b und 22b ab, sodass die Haltevorrichtung dann starr mit dem Stab 3b verbunden ist.

In Fig. 13 erkennt man schliesslich noch die Dreipunktauflage an den Auflagepunkten P1b,P2b,P3b. Eine derartige Dreipunktauflage ist konventionell, dazu muss die Ausnehmung in der Aufnahme 2b in dem unteren Bereich einen Radius aufweisen, der kleiner ist als der Radius des Stabs 3b. In den an diesen Bereich anschliessenden Bereichen muss der Radius der Ausnehmung dann grösser sein als der Radius des Stabs 3b. Dies erfolgt vorzugsweise mit einem tangentialen Übergang. Diese Art der Dreipunktauflage ist aber bereits für sich genommen bekannt.

Die Figuren 14 bis 17 zeigen ein weiteres Ausführungsbeispiel der erfindungsgemässen Haltevorrichtung. Man erkennt den Hakengrundkörper 1c, die Aufnahme 2c für den Stab 3c, den Deckel 6c und die Klemmschraube 5c, welche durch die Gewindebohrung 40c hindurch geschraubt werden kann. Der Deckel 6c weist zwei vom Deckel abstehende Flansche 60c auf, welche auf ihrer Innenwand jeweils einen nach innen abstehenden Zapfen 61c aufweisen. In der Wand der Aufnahme 2c ist eine Nut 21c vorgesehen, welche zunächst im wesentlichen in axialer Richtung verläuft und anschliessend im wesentlichen in Umfangsrichtung. Am oberen Ende weist die Nut 21c noch zwei Anschrägungen 210c auf, die das Einführen des Zapfens 61c in die Nut 21c hinein erleichtern. Der Deckel 6c wird in axialer Richtung auf den Hakengrundkörper 1c bzw. die Aufnahme 2c aufgeschoben, wobei die Zapfen 61c in axialer Richtung in die Nut 21c hinein gleiten. Ist der Deckel 6c soweit in axialer Richtung vorgeschoben worden, dass die Zapfen 61c am Ende des in axialer Richtung verlaufenden Teils der Nut 21c angelangt sind, kann der Deckel 6c relativ zum Hakengrundkörper 1c bzw. relativ zur Aufnahme 2c verdreht werden. Dadurch ist der Stab 3c in der Aufnahme 2c gefangen und kann nicht nach oben aus der Aufnahme 2c herausgleiten. Gleichwohl bleibt aber die Haltevorrichtung relativ zum Stab in axialer Richtung - bezogen auf den Stab - verschiebbar. Anschliessend wird die Klemmschraube 5c durch die Gewindebohrung 40c hindurch geschraubt und drückt den Stab 3c in die Aufnahme 2c hinein. Dabei wird der Deckel 6c nach oben gezogen, wobei sich die Zapfen 61c in der Nut 21c (in demjenigen Teil der Nut 21c, der in Umfangsrichtung verläuft) abstützen. Dadurch wird die Haltevorrichtung starr mit dem Stab 3c verbunden.

In Fig. 17 erkennt man schliesslich noch die Dreipunktauflage an den Auflagepunkten P1c,P2c,P3c. Eine derartige Dreipunktauflage ist konventionell, dazu muss die Ausnehmung in der Aufnahme 2c in dem unteren Bereich einen Radius aufweisen, der kleiner ist als der Radius des Stabs 3c. In den an diesen Bereich anschliessenden Bereichen muss der Radius der Ausnehmung dann grösser sein als der Radius des Stabs 3c. Dies erfolgt vorzugsweise mit einem tangentialen Übergang. Diese Art der Dreipunktauflage ist aber bereits für sich genommen bekannt.

Den Ausführungsbeispielen gemäss den Figuren 9 bis 13 und denen gemäss den Figuren 14 bis 17 ist gemeinsam, dass die Mittel zum Zurückhalten des Stabs 3b bzw. 3c in der Aufnahme 2b bzw. 2c einen Deckel 6b bzw. 6c umfassen, welcher so ausgebildet ist, dass er mit entsprechenden Mitteln der Haltevorrichtung eine Verbindung nach Art eines Bajonettverschlusses eingeht. Alternativen hierzu sind natürlich möglich und werden im folgenden noch beschrieben.

Die Figuren 18 bis 21 zeigen ein weiteres Ausführungsbeispiel der erfindungsgemässen Haltevorrichtung. Man erkennt den Hakengrundkörper 1d, die Aufnahme 2d für den Stab 3d, sowie einen Deckel 6d, welcher eine Gewindebohrung 40d aufweist, durch welche hindurch eine Klemmschraube 5d geschraubt werden kann. Man erkennt ferner, dass die Wandung der Aufnahme 2d in demjenigen Endbereich, welcher dem Deckel 6d zugewandt ist (also oben), elastisch deformierbar ausgebildet ist und Vorsprünge 22d aufweist. Ebenso weist der Deckel 6d Vorsprünge 62d auf (nämlich innen), welche mit den Vorsprüngen 22d der Aufnahme in Eingriff gebracht werden können. Zunächst wird der Hakengrundkörper 1d derart mit dem Stab 3d in Eingriff gebracht, dass der Stab 3d in die Aufnahme 2d eingeschnappt wird. Dadurch ist der Stab 3d gefangen und kann in axialer Richtung (also nach oben hin) nicht mehr aus der Aufnahme 2d herausgleiten. Sodann wird der Deckel 6d aufgeschoben. Dieser kann ungehindert auf die Aufnahme 2d aufgeschoben werden. Der in die Aufnahme eingeschnappte Stab ist in axialer Richtung - bezogen auf den Stab - noch verschiebbar. Die Klemmschraube 5d kann bereits lose in der Gewindebohrung 40d eingeschraubt sein, oder aber auch erst nachträglich eingeschraubt werden. Zur starren Befestigung der Haltevorrichtung an dem Stab 3d wird nun die Klemmschraube 5d eingeschraubt. Dabei deformieren sich die elastischen Endbereiche der Wandung mit den Vorsprüngen 22d nach aussen und gelangen auf diese Weise in Eingriff mit den Vorsprüngen 62d innen am Deckel (Fig. 20). Auf diese Weise wird die Haltevorrichtung dann starr mit dem Stab 3d verbunden.

In Fig. 21 erkennt man schliesslich noch die Dreipunktauflage an den Auflagepunkten P1d,P2d,P3d. Eine derartige Dreipunktauflage ist konventionell, dazu muss die Ausnehmung in der Aufnahme 2d in dem unteren Bereich einen Radius aufweisen, der kleiner ist als der Radius des Stabs 3d. In den an diesen Bereich anschliessenden Bereichen muss der Radius der Ausnehmung dann grösser sein als der Radius des Stabs 3d. Dies erfolgt vorzugsweise mit einem tangentialen Übergang. Diese Art der Dreipunktauflage ist aber bereits für sich genommen bekannt.

Schliesslich sind in den Figuren 22 bis 27 und in den Figuren 28 bis 33 noch zwei weitere Ausführungsbeispiele einer erfindungsgemässen Haltevorrichtung beschrieben. Beide weisen einen Hakengrundkörper 1e bzw. 1f auf, welcher eine Aufnahme 2e bzw. 2f aufweist. An der Aufnahme 2e bzw. 2f sind jeweils zwei hakenartig ausgebildete, feststehende Finger 21e und 22e bzw. 21f und 22f vorgesehen, welche zwischen sich eine Aussparung 23e bzw. 23f definieren. In dieser Aussparung 23e bzw. 23f ist jeweils ein schwenkbar bewegbarer Finger 24e bzw. 24f angeordnet. Bei den hakenartig ausgebildeten, feststehenden Fingern 21e und 22e bzw. 21f und 22f ist an der jeweiligen auf den beweglichen Finger zuweisenden Fläche ein Zapfen 210e und 220e bzw. 210f und 220f vorgesehen. Diese Zapfen greifen jeweils in eine entsprechende Aussparung 240e bzw. 240f (Fig. 25 bzw. Fig. 31) ein, welche an dem beweglichen Finger 24e bzw. 24f vorgesehen ist, und zwar jeweils in der entsprechenden Fläche, die auf den jeweiligen feststehenden Finger zuweist. Die Zapfen 210e und 220e bzw. 210f und 220f können dabei eingepresst und/oder angeschweisst bzw. geheftet sein.

Der schwenkbare Finger 24e bzw. 24f ist ferner noch mit einem Fortsatz 241e bzw. 241f versehen, welcher nach aussen über die Aussparung 23e bzw. 23f nach aussen hinaus steht. Dieser Fortsatz 241e bzw. 241f ist mit einem Aussengewinde versehen, auf welches eine Mutter 25e bzw. 25f aufschraubbar ist. In der geöffneten Stellung (Fig. 26 bzw. Fig. 32) kann der Stab 3e bzw. 3f in die Aufnahme 2e bzw. 2f eingeführt und anschliessend der schwenkbare Finger 24e bzw. 24f verschwenkt werden. Dadurch ist der Stab 3e bzw. 3f gefangen, weil die Zapfen in eine Hinterschneidung in der jeweiligen Aussparung 240e bzw. 240f des beweglichen Fingers 24e bzw. 24f hineingleiten. Gleichzeitig ist die Haltevorrichtung in axialer Richtung - bezogen auf den Stab 3e bzw. 3f - noch beweglich. Wird nun die Mutter 25e bzw. 25f angezogen, so wird der Stab 3e bzw. 3f durch den beweglichen Finger 24e bzw. 24f fest gegen den Stab 3e bzw. 3f gedrückt, der seinerseits fest in die Aufnahme 2e bzw. 2f gedrückt wird. Die Mutter 25e bzw. 25f stützt sich dabei ihrerseits auf den beiden feststehenden hakenartigen Fingern 21e und 22e bzw. 21f und 22f ab.

In den Figuren 27 bzw. 33 erkennt man noch die Dreipunktauflage mit den Auflagepunkten P1e,P2e,P3e bzw. P1f,P2f,P3f. Hierzu weist die Aufnahme 2e bzw. 2f in dem Bereich links einen Radius auf, der kleiner ist als der Radius des Stabs 3e bzw. 3f und in den an diesen Bereich anschliessenden Bereichen einen Radius, der grösser ist als der Radius des Stabs 3e bzw. 3f. Dies erfolgt vorzugsweise mit einem tangentialen Übergang. Diese Art der Dreipunktauflage ist jedoch für sich genommen bekannt.

Die Figuren 34 und 35 zeigen ein weiteres Ausführungsbeispiel der erfindungsgemässen Haltevorrichtung. Dieses Ausführungsbeispiel ist vom Prinzip her ähnlich wie das Ausführungsbeispiel, welches anhand der Figuren 14 bis 17 erläutert wurde. Bei dem in Fig. 34 bzw. in Fig. 35 gezeigten Ausführungsbeispiel erkennt man den Hakengrundkörper 1g, die Aufnahme 2g für den Stab 3g, den Deckel 6g sowie die Klemmschraube 5g, welche durch die Gewindebohrung 40g hindurch geschraubt werden kann. Am Hakengrundkörper 1g bzw. an der Aufnahme 2g sind Zapfen 21g vorgesehen, die von der Aussenwand der Aufnahme 2g nach aussen hin abstehen. Der Deckel 6g weist zwei vom Deckel abstehende Flansche 60g auf, in welchen jeweils eine Nut 61g vorgesehen ist. Die Nut 61g verläuft zunächst im wesentlichen in axialer Richtung und anschliessend im wesentlichen in Umfangsrichtung. An ihrem unteren Ende weist die Nut 61g zwei Anschrägungen 610g auf, die das Einführen der Zapfen 21g in die Nut 61g hinein erleichtern. Der Deckel 6g wird in axialer Richtung auf den Hakengrundkörper 1g bzw. auf die Aufnahme 2g aufgeschoben, wobei die Zapfen 21g in axialer Richtung in die Nut 61g hinein gleiten. Ist der Deckel 6g soweit in axialer Richtung vorgeschoben worden, dass die Zapfen 21g am Ende des in axialer Richtung verlaufenden Teils der Nut 61g angelangt sind, kann der Deckel 6g relativ zum Hakengrundkörper 1g bzw. relativ zur Aufnahme 2g verdreht werden. Dadurch ist der Stab 3g in der Aufnahme 2g gefangen und kann nicht nach oben aus der Aufnahme 2g herausgleiten. Gleichwohl bleibt aber die Haltevorrichtung relativ zum Stab in axialer Richtung - bezogen auf den Stab - verschiebbar. Anschliessend wird die Klemmschraube 5g durch die Gewindebohrung 40g hindurch geschraubt und drückt den Stab 3g in die Aufnahme 2g hinein. Dabei wird der Deckel 6g nach oben gezogen, wobei sich die Zapfen 21g in der Nut 61g (in demjenigen Teil der Nut 61g, der in Umfangsrichtung verläuft) abstützen. Dadurch wird die Haltevorrichtung starr mit dem Stab 3g verbunden. Auch hier erfolgt die bereits zuvor anhand der vorangehenden Ausführungsbeispiele erwähnte Dreipunktauflage des Stabs 3g.

Die Figuren 36 bis 43 zeigen weitere Ausführungsbeispiele des Deckels der erfindungsgemässen Haltevorrichtung, welche vom Prinzip her mit dem Ausführungsbeispiel gemäss den Figuren 5 bis 8 verwandt sind, nämlich derart, dass der Deckel mit dem Hakengrundkörper bzw. mit der Aufnahme eine Schnappverbindung eingeht. Der Hakengrundkörper kann im Prinzip so aussehen wie er in Fig. 8 dargestellt ist und ist daher nicht näher dargestellt.

In Fig. 36 ist ein Ausführungsbeispiel eines Deckels 6h dargestellt, der einen umlaufenden Flansch 60h aufweist, welcher vom Deckel absteht, in dem eine Gewindebohrung 40h für eine Klemmschraube vorgesehen ist. In dem Flansch sind zwei Aussparungen 61h für den Stab vorgesehen. Auf der Innenwand des Flanschs 60h sind Vorsprünge 62h und Vertiefungen 63h vorgesehen, welche mit entsprechenden Vorsprüngen bzw. Vertiefungen auf der Aussenwand der Aufnahme des Hakengrundkörpers die Schnappverbindung eingehen. Bei der Montage des Deckels 6h wird dieser in axialer Richtung über die Vorsprünge am Hakengrundkörper geschoben und schnappt ein. Der Flansch 60h muss eine gewisse Flexibilität aufweisen, sonst lässt er sich nicht über die Vorsprünge am Hakengrundkörper schieben. Um die Elastizität des Flansches 60h zu erhöhen, ist nahe dem Deckelboden eine umlaufende Aussparung 600h in der Innenwand des Deckels vorgesehen, welche in diesem Bereich die Wandstärke des Flansches 60h reduziert und so die erforderliche Elastizität des Deckels 6h erhöht.

Die Elastizität der Flansche 60i kann bei einer Weiterbildung des Deckels noch durch eine umlaufende Nut 610i auf der Aussenwand des Deckels 6i erhöht werden, wie dies in Fig. 37 und in Fig. 38 dargestellt ist, in welchen auch die Gewindebohrung 40i für ein Klemmschraube sowie die Aussparung 61 i für den Stab zu erkennen ist.

In Fig. 39 und Fig. 40 ist ein weiteres Ausführungsbeispiel des Deckels 6j mit der Gewinderbohrung 40j für eine Klemmschraube gezeigt, bei welchem nicht zwei, sondern vier Flansche 60j und dementsprechend auch vier Aussparungen für den Stab vorgesehen sind. Dadurch, dass nicht zwei, sondern vier solcher Flansche 60j und dementsprechend auch vier Aussparungen 61j für den Stab vorgesehen sind, werden die Flansche 60j von ihrer Ausdehnung in Umfangsrichtung kleiner und damit auch elastischer.

Im übrigen ist auch bei diesem Ausführungsbeispiel auf der Innenwand nahe dem Deckelboden eine umlaufende Aussparung 600j vorgesehen, wodurch die Elastizität der einzelnen Flansche 60j noch erhöht wird. Die Aussparungen 61j zwischen den Flanschen 60j haben alle die gleiche Breite b, sodass bei einem Stab mit entsprechendem Durchmesser der Deckel 6j in vier verschiedenen Winkelstellungen auf den Hakengrundkörper (nicht dargestellt) aufgeschoben werden kann, um die Schnappverbindung einzugehen.

In Fig. 41, Fig. 42 und Fig. 43 ist schliesslich ein weiteres Ausführungsbeispiel des Deckels 6k mit einer Gewindebohrung 40k für eine Klemmschraube dargestellt. Dieses Ausführungsbeispiel des Deckels weist ebenfalls vier Flansche 60k auf, zwischen denen Aussparungen 61k für den Stab vorgesehen sind. Wie speziell aus Fig. 42 und Fig. 43 zu erkennen ist, weisen jedoch die Aussparungen 61k nicht die gleiche Breite auf, sondern zwei sich gegenüber liegende Aussparungen 61k weisen eine erste Breite b1 auf (Fig. 42), während die beiden anderen sich gegenüber liegenden Aussparungen 61k eine zweite Breite b2 aufweisen (Fig. 43). Dies erhöht einerseits die Elastizität einzelner Flansche 60k, andererseits ist es auch möglich, Hakengrundkörper zu verwenden, die zwar einen einheitlichen Aussendurchmesser der Aufnahme aufweisen, bei denen jedoch die Innenabmessungen der Aufnahme variieren können, sodass je nach Innenabmessungen der Aufnahme des Hakengrundkörpers entweder ein Stab aufgenommen werden kann, dem die Breite b1 der Aussparung 61k des Deckels 6j entspricht, oder ein Stab, dem die Breite b2 der Aussparung 61k des Deckels 6j entspricht. Im übrigen ist auch hier auf der Innenwand des Deckels 6j nahe dem Deckelboden eine Aussparung 600k vorgesehen, welche die Elastizität der einzelnen Flansche 60k erhöht.

Wie bereits eingangs erwähnt, ist die erfindungsgemässe Haltevorrichtung, die anhand von verschiedenen Ausführungsbeispielen vorstehend erläutert ist, besonders für das Indikationsgebiet der Skoliose geeignet. Es ist aber ebenso auch geeignet für andere Indikationsgebiete wie z.B. Kyphose und Lordose. Die Haltevorrichtung kann ebenfalls so modifiziert sein, dass sie für anteriore Zugänge zur Wirbelsäule eingesetzt werden kann. Schliesslich kann die Haltevorrichtung auch so modifiziert sein, dass sie für die Querstabilisierung (zwischen den beiden Stäben) eingesetzt werden kann, sodass eine Torsion des Oberkörpers keine Bewegung der beiden Stäbe relativ zueinander hervorrufen kann.

## Patentansprüche

1. Haltevorrichtung für die Wirbelsäule, welche Haltevorrichtung Mittel zur Befestigung an einem Wirbel aufweist und welche eine Aufnahme (2) für einen Stab (3) aufweist, die an der den Befestigungsmitteln abgewandten Seite der Haltevorrichtung vorgesehen ist, sowie mit Mitteln (5) zum Herstellen einer starren Verbindung der Haltevorrichtung mit dem in der Aufnahme befindlichen Stab (3), dadurch gekennzeichnet, dass die Vorrichtung Mittel (4,20;6a,22a,62a;6b,22b,62;6c,21c,61c;6d,22d,62d; 21e,22e,24e;21f,22f,24f;6g,21g;61g;6h;6i;6j;6k) zum Zurückhalten des Stabes in der Aufnahme (2,2a,2b,2c,2d,2e,2f,2g) aufweist, welche so ausgebildet und mit der Haltevorrichtung derart in Verbindung bringbar sind, dass sie einen in die Aufnahme (2,2a,2b,2c,2d,2e,2f,2g) eingebrachten Stab (3,3a,3b,3c,3d,3e,3f,3g) vorläufig in der Aufnahme zurückhalten, bis die Haltevorrichtung mit Hilfe der Mittel zum Herstellen der starren Verbindung an dem Stab fixiert ist.

2. Haltevorrichtung nach Anspruch 1, dadurch gekennzeichnet, dass die Aufnahme für den Stab so ausgebildet ist, dass die Mittel zum Zurückhalten des Stabs (3,3a,3b,3c,3d,3e,3f,3g) mit der Aufnahme (2,2a,2b,2c,2d,2e,2f,2g) selbst in Verbindung bringbar sind.

3. Haltevorrichtung nach einem der vorangehenden Ansprüche, dadurch gekennzeichnet, dass die Mittel zum Zurückhalten des Stabs so ausgebildet sind, dass sie eine Klemmschraube (5,5a,5b,5c,5d,5g,25e,25f) aufnehmen, welche die Mittel zum Herstellen der starren Verbindung der Haltevorrichtung mit dem Stab bilden.

4. Haltevorrichtung nach einem der vorangehenden Ansprüche, dadurch gekennzeichnet, dass die Aufnahme für den Stab und die Mittel zum Herstellen der starren Verbindung der Haltevorrichtung mit dem Stab so ausgebildet sind, dass sie eine Dreipunktauflage (P1,P2,P3) bilden.

5. Haltevorrichtung nach einem der vorangehenden Ansprüche, dadurch gekennzeichnet, dass die Mittel zum Zurückhalten des Stabs ein Element (4,6a,6h,6i,6j,6k) umfassen, welches mit der Haltevorrichtung oder mit dem Stab eine Schnappverbindung eingeht.

6. Haltevorrichtung nach Anspruch 5, dadurch gekennzeichnet, dass das Element, welches die Schnappverbindung eingeht, durch einen zylindrischen, im Querschnitt sichelförmigen, Clip (4) gebildet ist, welcher so ausgebildet ist, dass er mit dem Stab eine Schnappverbindung eingeht, und dass die Aufnahme der Haltevorrichtung eine entsprechende zylindrische, im Querschnitt sichelförmige Aussparung (20) aufweist, welche von ihren Abmessungen geringfügig grösser ist als der Clip (4), sodass der auf die Stange aufgeschnappte Clip (4) in die Aussparung (20) in der Haltevorrichtung hinein eingeführt bzw. die Haltevorrichtung über den Clip (4) drüber geschoben werden kann.

7. Haltevorrichtung nach Anspruch 6, dadurch gekennzeichnet, dass der Clip (4) eine durchgehende Gewindebohrung (40) für eine Klemmschraube (5) aufweist, welche durch den Clip (4) hindurch schraubbar ist und den Stab (3) in der Aufnahme (2) verklemmt, sodass die Haltevorrichtung starr mit dem Stab (3) verbunden ist.

8. Haltevorrichtung nach Anspruch 5, dadurch gekennzeichnet, dass das Element, welches die Schnappverbindung eingeht, als Deckel (6a,6h,6i,6j.6k) mit einem vom Deckel abstehenden umlaufenden Flansch (60a,60h,60i,60j,60k) ausgebildet ist, in welchem Aussparungen (61 a,61 h,61 i,61j,61 k) für den Stab (3a) vorgesehen sind, und in welchem auf seiner Innenwand Vorsprünge (62a,62h) und Vertiefungen (63a,63h) vorgesehen sind, welche mit entsprechenden Vorsprüngen (22a) und Vertiefungen (23a) auf der Aussenwand der Aufnahme (2a) die Schnappverbindung eingehen.

9. Haltevorrichtung nach Anspruch 8, dadurch gekennzeichnet, dass der Deckel in seiner Stirnfläche mit einer durchgehenden Gewindebohrung (40a,40h,40i,40j,40k) versehen ist, durch welche eine Klemmschraube (5a) hindurch schraubbar ist, welche den Stab (3a) in der Aufnahme (2a) verklemmt, sodass die Haltevorrichtung starr mit dem Stab (3a) verbunden ist.

10. Haltevorrichtung nach einem der Ansprüche 1 bis 4, dadurch gekennzeichnet, dass die Mittel zum Zurückhalten des Stabs einen Deckel (6b,6c,6g) umfassen, welcher so ausgebildet ist, dass er mit entsprechenden Mitteln an der Haltevorrichtung eine Verbindung nach Art eines Bajonettverschlusses eingeht.

11. Haltevorrichtung nach Anspruch 10, dadurch gekennzeichnet, dass der Deckel (6b) zwei vom Deckel abstehende Flansche (60b) aufweist, die jeweils mit einer Anlagefläche (61 b) versehen sind, welche Anlagefläche (61 b) bei der Montage des Deckels (6b) an einer entsprechenden Anlagefläche (21 b) der Aufnahme (2b) entlang in axialer Richtung geführt wird, und dass der Deckel (6b) an demjenigen Ende der Anlagefläche (61 b), welches seiner inneren Stirnfläche zugewandt ist, mit einem in Umfangsrichtung verlaufenden Vorsprung (62b) versehen ist, welcher nach innen absteht und welcher beim Drehen des Deckels (6b) einen entsprechenden von der Aufnahme (2b) nach aussen abstehenden, ebenfalls in Umfangsrichtung verlaufenden Vorsprung (22b) hintergreift und so die Verbindung nach Art eines Bajonettverschlusses eingeht.

12. Haltevorrichtung nach Anspruch 10, dadurch gekennzeichnet, dass der Deckel (6c) zwei vom Deckel abstehende Flansche (60c) aufweist, welche auf ihrer Innenwand jeweils einen nach innen abstehenden Zapfen (61c) aufweisen, welcher in eine in der Wand der Aufnahme (2c) vorgesehene Nut (21c) eingreift und dort geführt ist, wobei die Nut (21c) zunächst im wesentlichen in axialer Richtung verläuft und anschliessend im wesentlichen in Umfangsrichtung, sodass bei der Montage des Deckels (6c) dieser zunächst in axialer Richtung geführt ist und anschliessend durch eine Drehbewegung die Verbindung nach Art eines Bajonettverschlusses eingeht.

13. Haltevorrichtung nach Anspruch 10, dadurch gekennzeichnet, dass auf der Wand der Aufnahme (2g) nach aussen hin abstehende Zapfen (21g) vorgesehen sind und der Deckel (6g) zwei vom Deckel abstehende Flansche (60g) aufweist, in denen jeweils eine Nut (61g) vorgesehen ist, in welche der jeweilige von der Wand der Aufnahme nach aussen hin abstehende Zapfen (21g) eingreift und in welcher er geführt ist, wobei die Nut (61g) zunächst im wesentlichen in axialer Richtung verläuft und anschliessend im wesentlichen in Umfangsrichtung, sodass bei der Montage des Deckels (6g) dieser zunächst in axialer Richtung geführt ist und anschliessend durch eine Drehbewegung die Verbindung nach Art eines Bajonettverschlusses eingeht.

14. Haltevorrichtung nach einem der Ansprüche 10 bis 13, dadurch gekennzeichnet, dass der Deckel (6b,6c,6g) in seiner Stirnfläche mit einer durchgehenden Gewindebohrung (40b,40c,40g) versehen ist, durch welche eine Klemmschraube (5b,5c) hindurch schraubbar ist, welche den Stab (3b,3c,3g) in der Aufnahme verklemmt, sodass die Haltevorrichtung starr mit dem Stab verbunden ist.

15. Haltevorrichtung nach einem der Ansprüche 1 bis 4, dadurch gekennzeichnet, dass die Mittel zum Zurückhalten des Stabs einen Deckel (6d) umfassen, welcher in axialer Richtung auf die Aufnahme (2d) aufschiebbar ist und welcher eine durchgehende Gewindebohrung (40d) aufweist, durch welche eine Klemmschraube (5d) hindurch schraubbar ist, und dass die Aufnahme (2d) in demjenigen Endbereich ihrer Wandung, welche dem Deckel (6d) zugewandt ist, elastisch deformierbar ausgebildet ist und Vorsprünge (22d) aufweist, welche nach einer Deformation mit entsprechend am Deckel (6d) ausgebildeten Vorsprüngen (62d) in Eingriff sind.

16. Haltevorrichtung nach einem der Ansprüche 1 bis 4, dadurch gekennzeichnet, dass die Mittel zum Zurückhalten des Stabs einen sichelförmigen Finger (24e,24f) umfassen, welcher in einer Aussparung (23e,23f) zwischen zwei an der Aufnahme hakenartig ausgebildeten, feststehenden Fingern (21e,22e;21f,22f) schwenkbar angeordnet ist, wobei an der jeweiligen auf den beweglichen Finger (24e,24f) zuweisenden Fläche der feststehenden hakenartigen Finger (21e,22e;21f,22f) jeweils ein auf den beweglichen Finger zuweisender Zapfen (210e,220e;210f,220f) vorgesehen ist, welcher in eine entsprechende Aussparung (240e,240f) eingreift, welche an dem beweglichen Finger (24e,24f) in der jeweiligen auf den feststehenden Finger (21e,22e;21f,22f) zuweisenden Fläche vorgesehen ist, und wobei der bewegliche Finger (24e,24f) einen nach aussen über die Aussparung hinaus stehenden Fortsatz (241e,241f) aufweist, der mit einem Gewinde versehen ist, auf welches eine Mutter (25e,25f) aufschraubbar ist.
